# EUROPÄISCHE PATENTSCHRIFT

(11) **EP 1 752 772 B1**
(45) Veröffentlichungstag und Bekanntmachung des Hinweises auf die Patenterteilung: **29.09.2010**
(21) Anmeldenummer: 06014877.2
(22) Anmeldetag: 18.07.2006
(51) Int. Cl.: G01N 33/86

(54) **Faktor Xa basiertes Heparin-Testverfahren unter Verwendung einer Heparin-modifizierenden Komponente**
Assay for determination of heparin based on factor Xa using a heparin-modifying component
Procédé pour détermination d'héparine basé sur facteur Xa utilisant un composant pour la modification d'héparine

(30) Priorität: 12.08.2005 DE 102005038418
(43) Veröffentlichungstag der Anmeldung: 14.02.2007
(73) Patentinhaber: Siemens Healthcare Diagnostics Products GmbH, 35041 Marburg (DE)
(72) Erfinder: Klein, Wolfgang, Dr., 35043 Marburg (DE); Zander, Norbert, Dr., 35039 Marburg (DE)

(56) Entgegenhaltungen:
- WO-A-02/23190
- WO-A-03/078960
- US-A- 4 234 682
- US-A- 5 702 912
- VAN PUTTEN J ET AL: "Determination of low molecular weight heparin in clinical laboratory" HAEMOSTASIS, BASEL, CH, Bd. 14, Nr. 2, 1984, Seiten 205-210, XP002903651

## Beschreibung

Die vorliegende Erfindung liegt auf dem Gebiet der Gerinnungsdiagnostik und betrifft ein in vitro-Verfahren zur Bestimmung der Heparinaktivität in einer Probe.

Heparine sind negativ geladene, polysulfatierte Glukosaminoglykanketten unterschiedlicher Länge und gehören zu den am weitesten verbreiteten gerinnungshemmenden Substanzen, die sofort und konzentrationsabhängig die Gerinnung in vivo und in vitro hemmen. Je nach Kettenlänge werden im wesentlichen sogenannte unfraktionierte, hochmolekulare Heparine (UFH, UF Heparine) mit einem Molekulargewicht zwischen 3 000 und 30 000 Dalton und sogenannte fraktionierte, niedermolekulare Heparine (LMWH, LMW Heparine) mit einem Molekulargewicht zwischen ca. 4 000 und 9 000 Dalton unterschieden. Fraktionierte Heparine werden im allgemeinen durch enzymatische oder chemische Spaltung oder chromatographisch aus unfraktioniertem Heparin gewonnen. Je nach Spaltungsmethode ergeben sich für diese Produkte unterschiedliche physiko-chemische Eigenschaften wie mittlere Kettenlänge, Molekulargewichtsverteilung, Sulfatierungsgrad oder Glykanmodifikationen, die einen Einfluss auf die biologische Aktivität dieser Produkte haben. Heparinderivate und Heparinoide sind enzymatisch und/oder chemisch veränderte Glukosaminoglykanketten, deren antikoagulatorische Eigenschaften durch die gezielte Beeinflussung von Glykankettenlänge, Sulfatierungs- bzw. Acetylierungsmuster oder die Mischung unterschiedlicher Glukosaminoglykane biotechnologisch modifiziert werden kann, wie z. B. bei Danaparoid-Natrium, einem Gemisch aus Glykosaminoglykanen, das überwiegend aus Heparansulfat und zu einem geringeren Anteil aus Dermatansulfat und Chondroitinsulfat besteht.

Die gerinnungshemmende Wirkung aller Heparine beruht auf ihrer Komplexbildung mit Antithrombin (AT, Antithrombin III), dem wichtigsten plasmatischen Inhibitor aktivierter Gerinnungsfaktoren. Antithrombin gehört zur Gruppe der Serinproteaseinihibitoren (Serpine) und hemmt die Gerinnungsfaktoren Thrombin (Faktor IIa, FIIa) und Faktor Xa (FXa) sowie in geringem Ausmaß auch die anderen Serinproteasen FIXa, FXIa, FXlla, Kallikrein und Plasmin. Durch die Bindung von Heparin an Antithrombin kommt es zu einer Konformationsänderung des Antithrombins, welche die inhibitorische Wirkung des Antithrombins um ein Vielfaches verstärkt. Die Bindungsstelle in Heparinmolekülen, die für die Bindung an Antithrombin verantwortlich ist, besteht aus einer charakteristischen Pentasaccharidsequenz. Eine vollständig synthetische Form dieses Pentasaccharids (Fondaparinux) wird ebenso wie UFH oder LMWH zur medikamentösen Hemmung der Gerinnungsfähigkeit eingesetzt.

Unfraktionierte Heparine und fraktionierte Heparine, Heparinderivate, Heparinoide bzw. Pentasaccharide unterscheiden sich in ihrer antikoagulatorischen Wirkung. Während UFH Thrombin und FXa gleichermaßen hemmen, weisen LMWH überwiegend eine FXa-hemmende Wirkung und nur in geringerem Maße eine Thrombin-hemmende Wirkung auf. Pentasaccharide wie Fondaparinux hemmen selektiv FXa und zeigen gar keine Thrombinhemmung.

Je nach Indikation werden Heparine prophylaktisch oder therapeutisch eingesetzt, z. B. zur Thromboembolieprophylaxe in Risikosituationen wie Operationen oder bei Immobilisierung, zur Therapie von bereits eingetretenen thromboembolischen Ereignissen oder auch bei extrakorporalen Therapieverfahren wie Hämodialyse, kardio-pulmonarem Bypass oder Herzkatheteruntersuchungen. Da der gerinnungshemmende Effekt von Heparin einer Reihe von individuellen Einflussgrößen, wie z. B. Körpergewicht oder Leber- und Nierenfunktion, unterliegt und da eine Fehldosierung Blutungskomplikationen bzw. thromboembolische Komplikationen auslösen kann, ist eine Therapieüberwachung für UFH bei allen und für LMWH bei Risikopatienten (Niereninsuffizienz, Übergewicht, Schwangerschaft) indiziert.

Im Stand der Technik sind verschiedene Verfahren zur Überwachung der Heparintherapie bzw. zur Bestimmung des Heparinspiegels bekannt.

Die Therapie und Prophylaxe mit Heparin kann zum einen indirekt überwacht werden, indem mit Hilfe von klassischen globalen Gerinnungstests, wie z. B. der PT, der APTT und der Thrombinzeit, die Gerinnungsfähigkeit des Blutes kontrolliert wird. Der Vergleich einer gemessenen Gerinnungszeit einer heparinisierten Patientenprobe mit entsprechenden Referenzwerten von Standardplasmen definierter Heparinkonzentration ermöglicht auch eine Quantifizierung des Heparinspiegels einer Patientenprobe. Beispiele für Gerinnungszeit-basierte Verfahren zur Bestimmung von Heparin sind z. B. in den Patentschriften US 4,067,777 und EP 217 768 B1 beschrieben.

Eine besondere Form einer Gerinnungszeit-basierten Heparinbestimmung ist in EP 259 463 B1 beschrieben. Bei diesem Verfahren wird für ein Aliquot einer Patientenprobe eine Gerinnungszeit bestimmt, während ein zweites Aliquot der Probe zum vollständigen Abbau des in der Probe enthaltenen Heparins mit Heparinase behandelt und dann erst die Gerinnungszeit bestimmt wird. Die Differenz der beiden Gerinnungszeiten ist ein Maß für die in der Probe enthaltene Heparinmenge, die an einer entsprechenden Standardkurve abgelesen werden kann. Allerdings werden in der Literatur globale Gerinnungstests als ungeeignet für die Bestimmung von LMWH betrachtet, da aufgrund deren verminderter Anti-Thrombinwirkung kein linearer, konzentrationsabhängiger Einfluss auf das Messergebnis (Gerinnungszeit, INR) besteht [siehe z. B. Harenberg, J. (2004) Is laboratory monitoring of low-molecular-weight heparin therapy necessary? Yes. J. Thromb. Haemost. 2; Seiten 547-550].

Zum anderen kann die Überwachung einer Heparintherapie oder -prophylaxe mit Hilfe sogenannter amidolytischer bzw. chromogener Heparinassays erfolgen, die eine präzise und sensitive Bestimmung der Heparinaktivität erlauben. Diese Verfahren basieren im wesentlichen darauf, dass eine heparinisierte Probe mit exogenem FXa oder Thrombin (FIIa) sowie einem entsprechenden, Faktorspezifischen chromogenen Substrat inkubiert wird und die Heparin-abhängige Inaktivierung der enzymatischen FXa- bzw. Thrombinaktivität anhand des umgesetzten Substrats ermittelt wird. Die Menge des freigesetzten chromogenen Spaltprodukts verhält sich umgekehrt proportional zur Heparinaktivität. Mit Hilfe einer Kalibrationskurve, erstellt aus der Messung von Plasmen mit bekannten Heparingehalten, lässt sich die Heparinmenge einer Patientenprobe korrekt quantifizieren. Die zusätzliche Zugabe von Antithrombin zum Reaktionsansatz, entweder in Form von Normalplasma oder auch in aufgereinigter Form, bewirkt einerseits durch die Schaffung eines Überschusses einen Ausgleich unterschiedlicher Antithrombin-Konzentrationen in unterschiedlichen Patientenproben und andererseits eine Erhöhung der Empfindlichkeit des Tests, insbesondere in Bereichen niedriger Heparinkonzentrationen. Derartige amidolytische Heparinassays sind in verschiedenen Varianten kommerziell erhältlich. Beispiele sind in den Patentschriften EP 004 271 A2 und EP 034 320 B1 beschrieben. Derartige chromogene Anti-FXa Teste sind nach dem Stand der Technik bei der Bestimmung von LMWH bevorzugt einzusetzen [siehe auch hier z. B. Harenberg, J. (2004)].

Weitere Verfahren sind in WO 03/078960 und WO 02/23190 beschrieben.

Die Quantifizierung von Heparin erfolgt üblicherweise in Internationalen Einheiten (Units) pro Milliliter (IU/ml). Da Heparine als extrahiertes biologisches Material starken Abhängigkeiten von Organismus, Gewebe, Extraktionsbedingungen u. a. unterliegen und die messbaren Effekte auf die Gerinnung nur indirekt erfasst werden können, hat sich eine physiko-chemische Wirkungsdefinition (Unit) als nicht praktikabel erwiesen. Es wurden daher entsprechende metrologische Standards definiert, gegen welche Präparate, aber auch neue Standards gemessen und deklariert werden. Am weitesten verbreitet ist das von der WHO unterstützte System der Internationalen Einheit (IU), welches vom National Institute for Biological Standards und Controls (NIBSC, UK) verwaltet wird [zur Übersicht siehe z. B. Mulloy, B. et al. (2000) Characterization of unfractionated heparin: Comparison of materials from the last 50 years. Thromb. Haemost. 84, 1052-1056 bzw. aktuellste Veröffentlichungen zu biologischen Standards auf der Homepage des NIBSC, www.nibsc.ac.uk].

Wie bereits oben beschrieben wurde, hemmen die unterschiedlichen Heparine die enzymatische Aktivität von Faktor Xa unterschiedlich stark. Aus diesem Grund muss für die Kalibrierung eines Faktor Xa-basierten Assays für jedes zu bestimmende Heparin eine eigene Kalibrationskurve erstellt werden, wobei unterschiedliche Konzentrationen desjenigen Heparins einzusetzen sind, welches in der Patientenprobe quantifiziert werden soll. Um eine präzise Heparinbestimmung einer Probe überhaupt durchführen zu können, ist es also notwendig zu wissen, mit welchem Heparinpräparat der Patient behandelt wurde, damit die gemessenen Werte an der richtigen Kalibrationskurve quantifiziert werden. Die Nachteile dieser Methode bestehen darin, dass die Erstellung verschiedener Kalibrationskurven Arbeits- und Materialkosten verursacht; dass immer die Gefahr falscher Angaben zum am Patienten verwendeten Heparinpräparat besteht, was die Benutzung einer falschen Kalibrationskurve zur Quantifizierung und infolgedessen eine falsche Interpretation der Testergebnisse bedingt, und dass, wenn keine Angaben darüber gemacht sind, mit welchem Präparat der Patient behandelt wurde, eine zuverlässige Heparinbestimmung nicht möglich ist.

Der vorliegenden Erfindung lag also die Aufgabe zugrunde, ein Verfahren zur Heparinbestimmung bereitzustellen, welches es ermöglicht, auf die Erstellung Heparinpräparat-spezifischer Kalibrationskurven zu verzichten und für alle Heparine eine universelle Kalibrationskurve zu verwenden. Die erfindungsgemäße Lösung der Aufgabe besteht in der Bereitstellung der in den Ansprüchen beschriebenen Gegenstände und Verfahren.

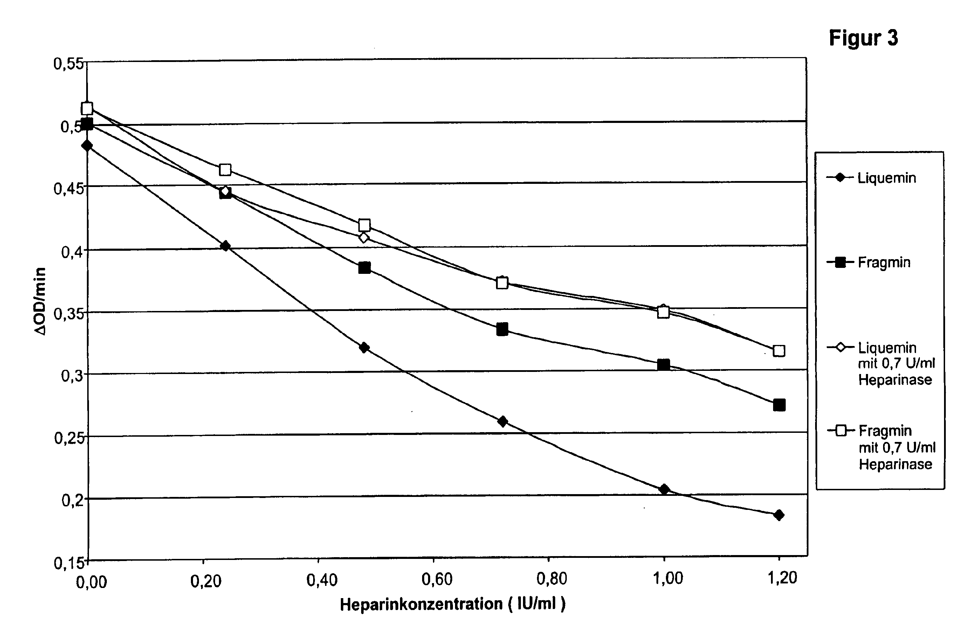

Die vorliegende Erfindung betrifft ein in vitro-Verfahren zur Bestimmung von Heparinen, d. h. von UF Heparin, LMW Heparin, Heparinderivaten, Heparinoiden und von Pentasacchariden in flüssigen Proben, insbesondere in Körperflüssigkeitsproben wie Blut, Plasma, Serum oder Urin, wobei die Heparinaktivität anhand der Heparin-abhängigen Faktor Xa-Inaktivierung bestimmt wird. Überraschenderweise wurde nun festgestellt, dass die Inkubation einer heparinisierten Probe, die unfraktioniertes oder fraktioniertes Heparin, ein Heparinderivat, ein Heparinoid oder ein synthetisches Pentasaccharid enthält, mit einer Heparin-modifizierenden Komponente dazu führt, dass die Unterschiede der FXa-inhibierenden Wirkung der unterschiedlichen Heparine ausgeglichen werden, die verbleibende FXa-inhibierende Wirkung jedoch weiterhin mit dem ursprünglich in der Probe vorhandenen Heparingehalt korreliert und dadurch die Quantifizierung an einer einzigen Kalibrationskurve möglich ist (siehe auch Figur 3).

Unter einer Heparin-modifizierenden Komponente im Sinne der vorliegenden Erfindung ist eine Substanz oder eine Mischung von Substanzen zu verstehen, die in der Lage ist, aus Heparin Oligosaccharide abzuspalten und die bei Verwendung in einem FXa-basierten Heparinsassay keine nachteiligen Effekte auf Bestandteile des Probenmaterials oder der anderen zugegeben Reagenzien ausübt, so dass eine quantitative Bestimmung der Heparin-abhängigen FXa-Inhibition in Anwesenheit der Substanz weiterhin möglich ist. Die Eignung einer Substanz zur Verwendung als Heparin-modifizierende Komponente im beschriebenen Verfahren kann z. B. ermittelt werden, indem ein FXa-basierter Heparinassay in Anwesenheit und parallel in Abwesenheit der zu untersuchenden Substanz durchgeführt wird. Geeignete Substanzen können daran erkannt werden, dass in UFH-enthaltenden Proben in Anwesenheit der Substanz eine geringere absolute Anti-FXa-Aktivität gemessen wird als in Abwesenheit der Substanz, wobei die in Anwesenheit der Substanz verbleibende Anti-FXa-Aktivität dennoch weiterhin mit der ursprünglich in der Probe enthaltenen Heparinaktivität korreliert, wodurch eine korrekte Quantifizierung der Heparinaktivität möglich ist.

Als Heparin-modifizierende Komponenten eignen sich für das erfindungsgemäße Verfahren Enzyme, da sie spezifisch und unter moderaten, nahezu physiologischen Bedingungen wirken, wodurch nachteilige Nebeneffekte auf das Testverfahren per se minimiert bzw. ausgeschlossen werden können. Besonders geeignet sind Enzyme aus den Gruppen der Glucuronidasen und Heparinasen, die über die Fähigkeit zur Verkürzung und Modifikation von Glykosaminoglykanketten verfügen. Glucuronidasen sind Heparin-hydrolysierende Enzyme verschiedenen Ursprungs, z. B. aus Escherichia coli, Rinderleber oder Mollusken, welche Glukosaminoglykanketten ohne chemische Modifikation verkürzen. Heparinasen, auch Heparin-Lyasen genannt, sind Heparin-spezifische Enzyme, die natürlicherweise von Mikroorganismen wie z. B. Flavobacterium heparinum synthetisiert werden und einen eliminativen Reaktionsmechanismus besitzen, also bei der Spaltreaktion eine endständige Zuckergruppe auch chemisch modifizieren. Zur Verwendung als Heparin-modifizierende Komponente im erfindungsgemäßen Verfahren eignen sich insbesondere Heparinase I, Heparinase II und Heparinase III, welche entweder direkt aus z. B. F. heparinum Kulturen gewonnen oder auch rekombinant z. B. in Escherichia coli exprimiert werden können. Ebenso geeignet sind Mischungen verschiedener Heparinasen und/oder Glucuronidasen als Heparin-modifizierende Komponente. Verfahren zur Gewinnung der endogenen Heparinasen aus F. heparinum sowie zur gentechnischen Herstellung der Enzyme sind im Stand der Technik beschrieben, z. B. in US 5,714,376 und in EP 670 892 B1 und den darin zitierten Publikationen.

Während im Stand der Technik Heparin-modifizierende bzw. -abbauende Enzyme dazu verwendet werden, Heparin durch vollständigen Abbau zu neutralisieren, werden die Heparin-modifizierenden Komponenten im vorliegenden Verfahren so eingesetzt, dass sie das in einer Probe enthaltene Heparin nicht vollständig abbauen, sondern dass das in der Probe enthaltene Heparin lediglich so modifiziert wird, dass eine Quantifizierung der Antithrombin-aktivierenden Eigenschaften weiterhin möglich ist. Dazu wird die Heparin-modifizierende Komponente mit der Probe bevorzugt in einer Konzentration von 0,05 bis 5,0 U/ml, besonders bevorzugt in einer Konzentration von 0,25 bis 1,25 U/ml im Ansatz inkubiert, bevor FXa zugegeben wird. Dabei entspricht 1 Unit der Enzymmenge, die benötigt wird, um aus Heparin (aus Schweinemukosa; ca. 109 IU/mg) bei 30 °C und pH 7,0 pro Minute 1 µmol ungesättigter Oligosaccharide abzuspalten.

Bei dem vorliegenden Verfahren wird die Probe mit der Heparin-modifizierenden Komponente für einen definierten Zeitraum inkubiert, bevor Faktor Xa zugegeben wird. Bevorzugterweise wird die Probe vor der Zugabe von FXa für eine Dauer von 10 bis 900 Sekunden, besonders bevorzugt für eine Dauer von 15 bis 90 Sekunden, ganz besonders bevorzugt für eine Dauer von 20 bis 60 Sekunden mit einer Heparin-modifizierenden Komponente inkubiert. Kürzere sowie längere Inkubationszeiten sind möglich, wobei die Dauer der Inkubationszeit in erster Linie mit der eingesetzten Konzentration der Heparin-modifizierenden Komponente und der gewählten Reaktionstemperatur abzustimmen ist. Üblicherweise wird zur Bestimmung physiologisch relevanter Parameter in der Gerinnungsdiagnostik eine Reaktionstemperatur von 37 °C bevorzugt. Niedrigere oder höhere Reaktionstemperaturen können ebenfalls verwendet werden, sofern der Fachmann die ihm bekannten thermodynamischen Auswirkungen, z. B. auf die Kinetik biochemischer Reaktionen, einkalkuliert.

In einer bevorzugten Ausführungsform des erfindungsgemäßen Verfahrens wird eine Citratplasmaprobe zunächst mit einem Reagenz vermischt, das Antithrombin enthält. Es kann sich dabei z. B. um Normalplasma oder auch um aufgereinigtes Antithrombin handeln. Bevorzugterweise wird Antithrombin im Überschuss zugegeben, so dass mehr Antithrombin im Ansatz vorhanden ist, als für die Inhibierung von FXa notwendig ist. Für den Fall, dass die Probe mit einem Antithrombin-Reagenz vorbehandelt wird, kann die Heparin-modifizierende Komponente bevorzugt in diesem Reagenz enthalten sein.

Nach der Inkubation der Probe mit der Heparin-modifizierenden Komponente und gegebenenfalls mit Antithrombin wird dem Reaktionsansatz Faktor Xa zugegeben. Es kann sich dabei um humanen, bovinen oder eine rekombinante Variante des Faktor Xa handeln. In einer besonders bevorzugten Auführungsform kann das FXa-Reagenz zusätzlich Dextransulfat enthalten. Es ist ebenso möglich, das Dextransulfat unabhängig vom FXa-Reagenz zur Probe zu geben.

Nach einer Inkubationsphase, während der der zugegebene Faktor Xa in Abhängigkeit von dem in der Probe enthaltenen Heparin bzw. dem gemäß dieser Erfindung modifizierten Heparinderivat inaktiviert wird, wird dem Reaktionsansatz ein Substrat zugegeben. Bevorzugterweise werden chromogene Substrate verwendet, die unter Einwirkung von Faktor Xa einen Farbstoff abspalten. Dabei kann es sich um einen im sichtbaren Bereich des Spektrums bestimmbaren Farbstoff, einen Fluoreszenzfarbstoff oder einen im UV-Bereich bestimmbaren Farbstoff handeln. Bevorzugterweise werden Peptide verwendet, die an der Carboxygruppe eines Argininrests einen durch Amidbindung gebundenen Farbstoffrest aufweisen. Besonders geeignet sind hierfür p-Nitroanilidgruppen (pNA) und 5-Amino-2-Nitro-Benzoesäurederivate (ANBA), sowie von diesen durch Substitution abgeleitete Farbstoffe, welche nach Abspaltung vom Peptidanteil durch eine photometrische Messung bei 405 nm Wellenlänge quantifiziert werden können. Beispiele für geeignete Substrate sind Bz-Ile-Glu-Gly-Arg-pNA und Z-D-Leu-Gly-Arg-ANBA-methylamid.

Das Verfahren kann kinetisch oder als Endpunktbestimmung ausgewertet werden. Bei der kinetischen Methode wird die Reaktion, d. h. die verbliebene Faktor Xa-Aktivität als Maß für die vorhandene Heparinaktivität, anhand der Umsatzrate des chromogenen Substrats quantifiziert. Bei der Endpunktbestimmung wird nach einer vorbestimmten Messzeit die Reaktion durch Zugabe einer Säure, z. B. von Essigsäure, gestoppt und die Menge des freigesetzten Farbstoffs gemessen.

In einer bevorzugten Ausführungsform des erfindungsgemäßen Verfahrens wird die Heparinaktivität einer Probe unabhängig von der Art des in der Probe enthaltenen Heparins anhand einer universellen, für alle Heparinvarianten geeigneten Kalibrationskurve quantifiziert. Die Auswertung erfolgt anhand einer Bezugskurve, die mit Hilfe einer Kalibratorlösung, die eine definierte Menge Heparin enthält, und/oder mit Hilfe von Verdünnungen dieser Kalibratorlösung erstellt wurde. Im Gegensatz zu den im Stand der Technik bekannten Verfahren ist es für das erfindungsgemäße Verfahren nicht erforderlich, dasselbe Heparin-Präparat zur Kalibration zu verwenden, welches in einer Patientenprobe bestimmt werden soll. Das erfindungsgemäße Verfahren erlaubt es, jedes beliebige Heparin, unfraktioniertes oder fraktioniertes Heparin oder ein Pentasaccharid, zur Kalibration zu verwenden und die Heparinaktivität jeder Patientenprobe unabhängig davon, welches Präparat verabreicht wurde, an der erstellten Kalibrationskurve zu quantifizieren. Zum Beispiel können Liquemin^{®} (Roche, Basel, Schweiz), ein unfraktioniertes, hochmolekulares Heparin aus Schweinemukosa, oder Fragmin^{®} (Pharmacia, Kalamazoo, USA), ein fraktioniertes, niedermolekulares Heparin, zur Erstellung einer universellen, für alle Heparinvarianten geeigneten Kalibrationskurve verwendet werden. Bevorzugterweise wird ein UF Heparin oder ein LMW Heparin mit isotonischer Kochsalzlösung auf 10 IU/ml verdünnt und schließlich mit einem heparinfreien Pool- oder Standardplasma verdünnt, so dass mit dem erfindungsgemäßen Verfahren eine Bezugskurve für den gesamten therapeutischen Bereich erstellt werden kann.

Das erfindungsgemäße Verfahren eignet sich sowohl zur manuellen Durchführung wie auch zur Anwendung in automatischen Analysegeräten.

Das erfindungsgemäße Verfahren eignet sich nur bedingt zur Bestimmung der Heparinaktivität in einer Probe, die zusätzlich eine Substanz enthält, die die bei Durchführung des Verfahrens verwendete Heparin-modifizierende Komponente inhibiert. Das Patentdokument WO 01/46385 A1 beschreibt z. B. die Verbindungen CRM646-A und CRM646-B, die als Heparinase-Inhibitoren wirken und die für eine therapeutische Verwendung geeignet scheinen. Bei der erfindungsgemäßen Bestimmung der Heparinaktivität in einer Probe, die eine Substanz enthält, die die verwendete Heparin-modifizierende Komponente inhibiert, könnte die Heparin-modifizierende Wirkung beeinträchtigt werden. Eine Quantifizierung des Heparingehalts einer solchen Probe an einer zuvor mit Hilfe des erfindungsgemäßen Verfahrens erstellten Kalibrationskurve, würde höchstwahrscheinlich zu Fehlbestimmungen führen. Das erfindungsgemäße Verfahren eignet sich demnach bevorzugt zur Bestimmung der Heparinaktivität in einer flüssigen Probe, die frei ist von Substanzen, die die im Verfahren verwendete Heparin-modifizierende Komponente inhibieren. In bevorzugten Ausführungsformen ist die Probe frei von Glucuronidase-Inhibitoren und/oder frei von Heparinase-Inhibitoren, wie z. B. CRM646-A und CRM646-B (WO01/46385 A1). Proben, die im Sinne der vorliegenden Erfindung frei sind von Heparinase- und/oder Glucuronidase-Inhibitoren, sind z. B. Körperflüssigkeitsproben, die von einem oder mehreren Menschen oder Tieren stammen, die weder in vivo mit einem solchen Inhibitor therapeutisch behandelt wurden noch in vitro mit einem solchen Inhibitor versetzt wurden.

Ein weiterer Aspekt der vorliegenden Erfindung betrifft ein Testkit zur Durchführung des erfindungsgemäßen FXa-basierten Heparinassays, wobei das Testkit mindestens zwei Reagenzien enthält, von denen eines eine Heparin-modifizierende Komponente wie in Anspruch 11 definiert enthält und das andere FXa. Die Reagenzien können zusätzlich Konservierungsmittel enthalten und entweder als Flüssigreagenzien oder als Lyophilisate bereitgestellt werden.

Das Reagenz, das die Heparin-modifizierende Komponente enthält, kann zusätzlich eine weitere Komponente enthalten, die zur Durchführung eines FXa-basierten Heparinassays benötigt wird, wie z. B. das Antithrombin oder ein Substrat. Je nach Testdesign kann sich die Reihenfolge der Zugabe der einzelnen Komponenten zur Probe unterscheiden. In einigen Fällen wird die Nachweisreaktion durch Zugabe des Substrats gestartet, in anderen Fällen durch Zugabe von FXa. Zu beachten ist, dass die Heparin-modifizierende Komponente in einem Reagenz enthalten ist, welches vor der Zugabe von FXa mit der Probe vermischt wird. In einer besonders bevorzugten Ausführungsform eines erfindungsgemäßen Testkits ist ein Reagenz enthalten, dass die Heparin-modifizierende Komponente zusammen mit Antithrombin enthält.

Neben dem Reagenz, das die Heparin-modifizierende Komponente enthält und dem Reagenz, das FXa enthält, kann ein erfindungsgemäßes Testkit außerdem weitere Reagenzien enthalten, wie z. B. ein Reagenz, das ein Faktor Xa-Substrat enthält, und/oder ein Reagenz, das Antithrombin enthält. Ein bevorzugtes FXa-Reagenz kann zusätzlich Dextransulfat enthalten. Ein anderes bevorzugtes Reagenz kann Antithrombin zusammen mit Dextransulfat enthalten.

Die Reagenzien des erfindungsgemäßen Testkits können in flüssiger oder lyophilisierter Form bereitgestellt werden. Für den Fall, dass einige oder alle Reagenzien des Testkits als Lyophilisate vorliegen, kann das Testkit zusätzlich die zur Lösung der Lyophilisate erforderlichen Lösemittel enthalten, wie z. B. destilliertes Wasser, geeignete Puffer und/oder Standard-Human-Plasma.

Die folgenden Ausführungsbeispiele dienen der Veranschaulichung des erfindungsgemäßen Verfahrens und sind nicht als Einschränkung zu verstehen.

### Beispiele

### Beispiel 1

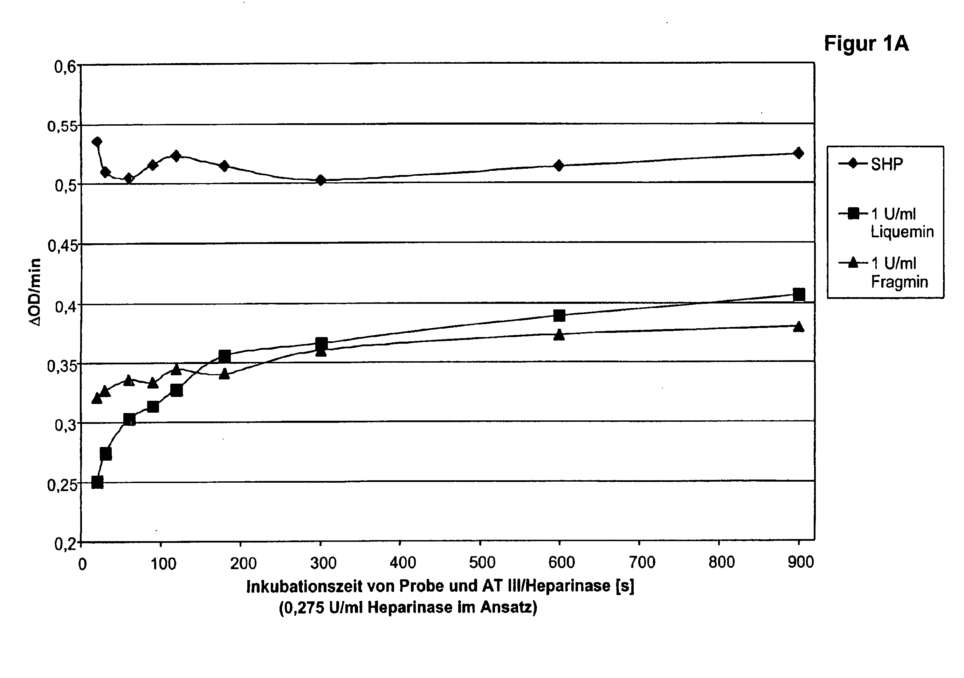

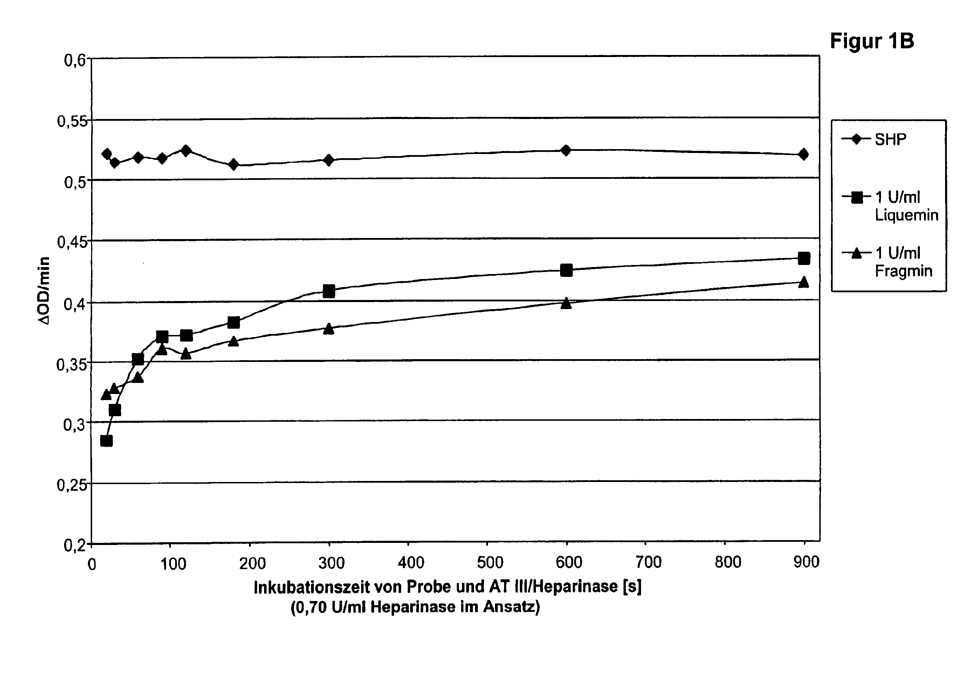

Lyophilisiertes und rekonstituiertes humanes Citratplasma wurde mit Liquemin^{®} (Roche, Basel, Schweiz), einem UF Heparin bzw. mit Fragmin^{®} (Pharmacia, Kalamazoo, USA), einem LMW Heparin, auf 1 IU/ml aufgestockt. Jeweils 20 µl Plasma wurden mit 20 µl Antithrombin-Reagenz (AT-Reagenz; lyophilisiert und rekonstituiert, 1 IU/ml), welches mit 0,55 U/ml (Fig. 1A) bzw. 1,4 U/ml (Fig. 1 B) Heparinase I (Dade^{®} Hepzyme^{®}, Dade Behring Marburg GmbH, Marburg, Deutschland) versetzt war, für 20 bis 900 Sekunden bei 37 °C inkubiert. Danach wurde der Reaktionsansatz mit 170 µl Faktor Xa-Reagenz [lyophilisierter und rekonstituierter humaner FXa mit Zusatz von TRIS (6 g/l), EDTA (0,74 g/l) und Natriumchlorid (12 g/l)] gemischt und für 1 Minute bei 37 °C inkubiert. Durch Zugabe von 40 µl Substratreagenz (Z-D-Leu-Gly-Arg-ANBA-methylamid, 4 mM) wurde die Reaktion gestartet und die Bildung von ANBA bei 405 nm Wellenlänge und 37 °C auf einem automatischen Messgerät, dem Sysmex^{®} CA-1500 (Dade Behring Marburg GmbH, Marburg, Deutschland) erfasst. Die Extinktionsänderung pro Minute (ΔOD/min) wurde für die unterschiedlichen Inkubationszeiten von Plasma mit AT-Heparinase-Reagenz in Figur 1 dargestellt.

Figur 1 zeigt den Verlauf der Messsignale (ΔOD/min) gegen die Inkubationszeit bei zwei unterschiedlichen Heparinaseaktivitäten im Ansatz für Plasmen mit UFH (Liquemin^{®}) bzw. LMWH (Fragmin^{®}). Als Kontrolle wurde jeweils ein Heparin-freies Standardhumanplasma (SHP) gemessen. Dieses Plasma ohne Heparin zeigt keine Extinktionsänderungen in Abhängigkeit von der Inkubationszeit der Probe mit AT-Heparinasereagenz. Bei den Heparin-haltigen Plasmen steigt mit zunehmender Inkubationszeit die Extinktionsänderung. Nach ca. 300 Sekunden erreicht die Extinktionsänderung ein Plateau, welches deutlich unterhalb dem des Heparin-freien Plasmas liegt. Auch eine auf 900 Sekunden verlängerte Inkubation der Probe mit Heparinase kann die Anti-FXa Aktivität der untersuchten Heparine nicht vollständig eliminieren. Die hier eingesetzten Heparinasegehalte modulieren nur in geringem Umfang die absolute Höhe des erreichten Plateaus, vergleiche Figur 1A (0,275 U/ml Heparinase im Ansatz vor Zugabe von FXa) und Figur 1B (0,7 U/ml Heparinase im Ansatz vor Zugabe von FXa).

### Beispiel 2

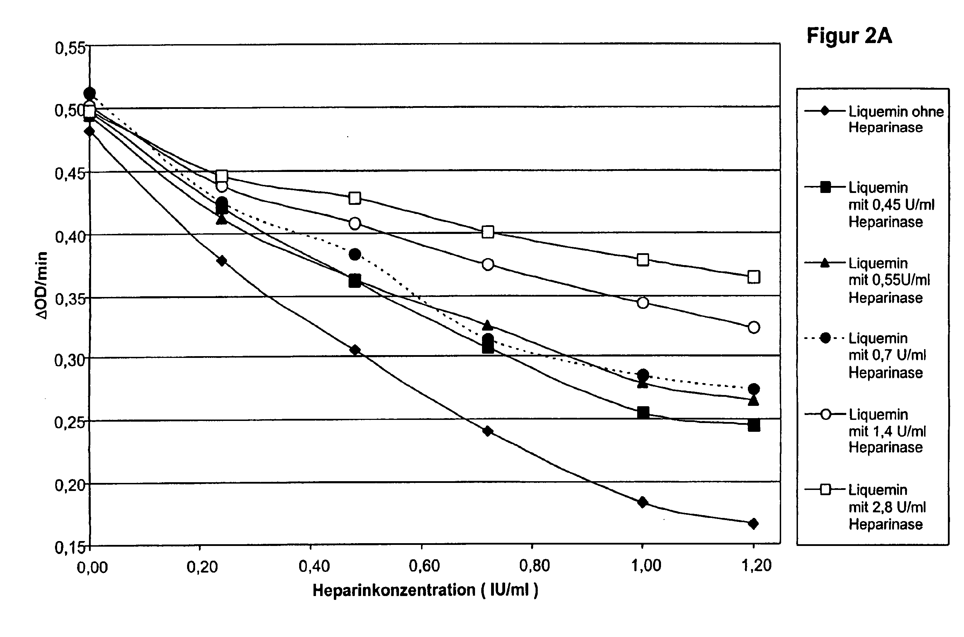

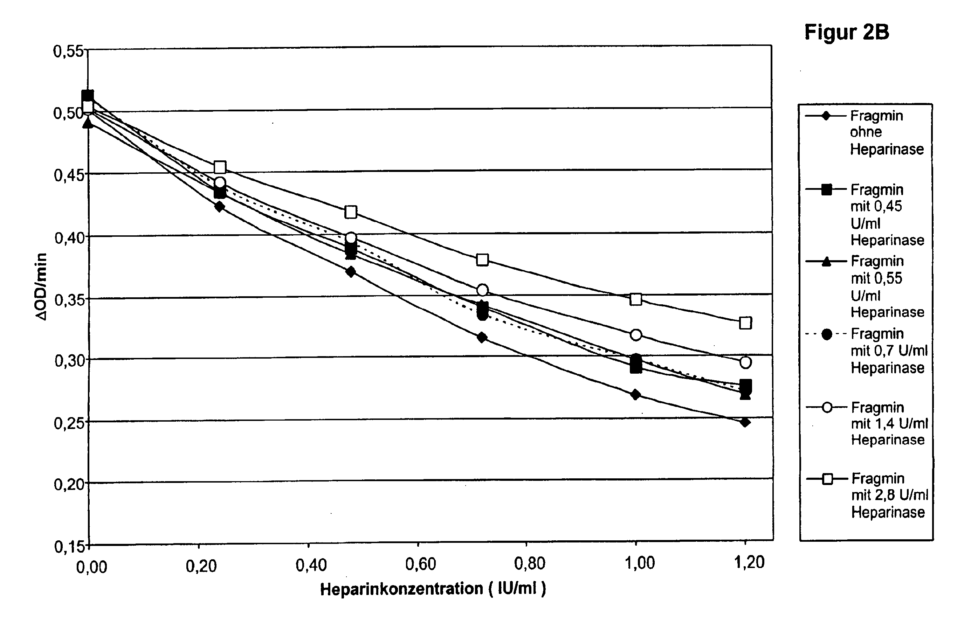

Lyophilisiertes und rekonstituiertes humanes Citratplasma wurde mit Liquemin^{®} (Figur 2A) bzw. Fragmin^{®} (Figur 2B) jeweils auf 0,24; 0,48; 0,72; 1 und 1,2 IU/ml aufgestockt. AT-Reagenz (lyophilisiert und rekonstituiert, 1 IU/ml) wurde mit steigenden Konzentrationen Heparinase I (Dade^{®} Hepzyme^{®}) von 0 bis 5,5 U/ml versetzt. Jeweils 20 µl Plasma wurden mit jeweils 20 µl AT-Heparinase-Reagenz gemischt und für 20 Sekunden bei 37 °C inkubiert. Danach wurden die Reaktionsansätze mit 170 µl Faktor Xa-Reagenz (siehe Beispiel 1) gemischt und für 1 Minute bei 37 °C inkubiert. Durch Zugabe von 40 µl Substratreagenz (siehe Beispiel 1) wurden die Reaktionen gestartet und die Bildung von ANBA, wie bereits in Beispiel 1 beschrieben, automatisch erfasst. Die Extinktionsänderung pro Minute (ΔOD/min) für die unterschiedlichen Reaktionsansätze von Plasma mit AT-Heparinase-Reagenz ist in Figur 2 dargestellt. Auf diese Art wurden Kalibrationskurven bei unterschiedlichen Heparinaseaktivitäten simuliert.

In Figur 2 ist der Verlauf der Messsignale (ΔOD/min) gegen verschiedene Heparinkonzentrationen in Citratplasma dargestellt. Die unterschiedlichen Kurven geben die unterschiedlichen Heparinaseaktivitäten im Ansatz vor Zugabe von FXa wieder. In Figur 2A wurden die Plasmen mit UFH (Liquemin^{®}) aufgestockt, in Figur 2B mit LMWH (Fragmin^{®}). Die Abbildungen zeigen, dass die Kalibrationskurven sowohl für UFH's als auch für LMWH's mit steigenden Heparinasegehalten flacher werden, andererseits bei gleichen Heparingehalten bzw.- aktivitäten von Liquemin^{®} oder Fragmin^{®} die initiale Abbaugeschwindigkeit für UF Heparin (Liquemin^{®}) größer als für LMW Heparin (Fragmin^{®}) ist. Auch bei der höchsten im Beispiel dargestellten Heparinasekonzentration ergibt sich ein linear monotoner, von der Ausgangskonzentration der Heparine abhängender Verlauf der Kalibrationsgeraden. Die Anti-FXa-Aktivität der Heparine wird auch bei einer Heparinase-Inkubationsdauer von 20 Sekunden bei 37 °C durch die höchste im Beispiel gewählte Heparinaseaktivität nicht vollständig eliminiert.

### Beispiel 3

Hier wurde ein exemplarischer Testaufbau gewählt, um das Potenzial einer gleichmäßigen Modifikation von UFH und LMWH sowie die Möglichkeit einer gemeinsamen Kalibrationskurve zu verdeutlichen.
Lyophilisiertes und rekonstituiertes humanes Citratplasma wurde mit Liquemin^{®} bzw. Fragmin^{®} jeweils auf 0,24; 0,48; 0,72; 1,00 und 1,20 IU/ml aufgestockt. AT-Reagenz (lyophilisiert und rekonstituiert, 1 IU/ml) wurde mit Heparinase I (Dade^{®} Hepzyme^{®}) (1,4 U/ml) versetzt, als Kontrolle diente AT-Reagenz ohne Heparinasezusatz. Jeweils 20 µl Plasma wurden mit jeweils 20 µl AT Reagenz gemischt und für 20 Sekunden bei 37 °C inkubiert. Danach wurden die Reaktionsansätze mit 170 µl Faktor Xa-Reagenz (siehe Beispiel 1) gemischt und für 1 Minute bei 37 °C inkubiert. Durch Zugabe von 40 µl Substratreagenz (siehe Beispiel 1) wurden die Reaktionen gestartet und die Bildung von ANBA, wie bereits in Beispiel 1 beschrieben, automatisch erfasst. Die Extinktionsänderung pro Minute (ΔOD/min) wurde für die unterschiedlichen Reaktionsansätze von Plasma mit AT-Heparinase-Reagenz in Figur 3 dargestellt.

In Figur 3 ist der Verlauf der Messsignale (ΔOD/min) gegen verschiedene Heparinkonzentrationen in Citratplasma dargestellt. Die Kurven beschreiben jeweils eine Kombination von Heparin (UFH oder LMWH) und einer gewählten Heparinaseaktivität (0 U/ml als Basistest, entsprechend dem Stand der Technik, und 0,7 U/ml, entsprechend der hier beschriebenen Erfindung). Figur 3 zeigt die Divergenz von UFH und LMWH Kalibrationskurven ohne Heparinasezusatz an den Beispielen Liquemin^{®} und Fragmin^{®} (gefüllte Symbole). Die Durchführung der Kalibrationen mit Heparinase-haltigem AT-Reagenz führt dazu, dass die Kalibrationskurven der verschiedenen Heparine in ihrem Verlauf zusammenrücken (offene Symbole). Auch wenn die Steigung der Kalibrationskurven in dem gezeigten Beispiel durch den Einsatz der Heparin-modifizierenden Komponenten geringer werden, so besteht nach wie vor ein eindeutiger linearer Zusammenhang zwischen den Messsignalen und der Heparinkonzentration im Plasma, welcher die korrekte Heparinkonzentrationsbestimmung von Plasmaproben anhand dieser Kalibrationskurven erlaubt.

Durch seine allgemeine Fachkenntnis ist der Fachmann in der Lage, durch routinemäßige Modifikationen in der Testabarbeitung (z. B. Änderung des Probenvolumens, Änderung der FXa-Inaktivierungszeit, Änderung der Reaktionstemperatur, des pH-Wertes, der lonenstärke oder anderer Parameter), die Steigung der Kalibrationskurve zu verändern und an die Testanforderungen anzupassen.

## Patentansprüche

1. Verfahren zur Bestimmung der Heparinaktivität in einer flüssigen Probe, wobei die Heparin-abhängige Inaktivierung von zugesetztem Faktor Xa quantifiziert wird, **dadurch gekennzeichnet, dass** die Probe vor der Zugabe von Faktor Xa mit einem Enzym aus der Gruppe der Heparinasen oder mit einem Enzym aus der Gruppe der Glucuronidasen oder mit einer Mischung verschiedener Enzyme aus einer der Gruppen oder mit einer Mischung verschiedener Enzyme aus beiden Gruppen inkubiert wird.

2. Verfahren gemäß Anspruch 1 **dadurch gekennzeichnet, dass** die Probe vor der Zugabe von Faktor Xa mit Heparinase I, Heparinase II, Heparinase III oder einer Mischung davon inkubiert wird.

3. Verfahren gemäß einem der Ansprüche 1 bis 2 **dadurch gekennzeichnet, dass** die Probe vor der Zugabe von Faktor Xa mit einem Enzym aus der Gruppe der Heparinasen oder mit einem Enzym aus der Gruppe der Glucuronidasen oder mit einer Mischung verschiedener Enzyme aus einer der Gruppen oder mit einer Mischung verschiedener Enzyme aus beiden Gruppen in einer Konzentration von 0,05 bis 5,0 U/ml, besonders bevorzugt in einer Konzentration von 0,25 bis 1,25 U/ml im Ansatz inkubiert wird.

4. Verfahren gemäß einem der Ansprüche 1 bis 3 **dadurch gekennzeichnet, dass** die Probe vor der Zugabe von Faktor Xa für 10 bis 900 Sekunden, bevorzugt für 15 bis 90 Sekunden, besonders bevorzugt für 20 bis 60 Sekunden mit einem Enzym aus der Gruppe der Heparinasen oder mit einem Enzym aus der Gruppe der Glucuronidasen oder mit einer Mischung verschiedener Enzyme aus einer der Gruppen oder mit einer Mischung verschiedener Enzyme aus beiden Gruppen inkubiert wird.

5. Verfahren gemäß einem der Ansprüche 1 bis 4 **dadurch gekennzeichnet, dass** die Heparin-abhängige Inaktivierung von zugesetztem Faktor Xa in Anwesenheit von zugesetztem Antithrombin quantifiziert wird.

6. Verfahren gemäß Anspruch 5 **dadurch gekennzeichnet, dass** das Antithrombin gleichzeitig mit einem Enzym aus der Gruppe der Heparinasen oder mit einem Enzym aus der Gruppe der Glucuronidasen oder mit einer Mischung verschiedener Enzyme aus einer der Gruppen oder mit einer Mischung verschiedener Enzyme aus beiden Gruppen zur Probe gegeben wird.

7. Verfahren gemäß einem der Ansprüche 1 bis 6 **dadurch gekennzeichnet, dass** zusätzlich Dextransulfat zur Probe gegeben wird.

8. Verfahren gemäß Anspruch 7 **dadurch gekennzeichnet, dass** das Dextransulfat gleichzeitig mit dem Faktor Xa zur Probe gegeben wird.

9. Verfahren gemäß einem der Ansprüche 1 bis 8 **dadurch gekennzeichnet, dass** die Heparinaktivität in einer Körperflüssigkeitsprobe bestimmt wird, bevorzugt in Blut, Blutplasma, Serum oder Urin.

10. Verfahren gemäß einem der Ansprüche 1 bis 9 weiterhin **dadurch gekennzeichnet, dass** unabhängig von der Art des in der Probe enthaltenen Heparins die Heparinaktivität in der Probe anhand einer universellen, für alle Heparinvarianten geeigneten Kalibrationskurve quantifiziert wird, die zuvor unter Verwendung eines beliebigen Heparins erstellt wurde.

11. Testkit zur Durchführung des Verfahrens gemäß Anspruch 1 **dadurch gekennzeichnet, dass** ein Reagenz, enthaltend ein Enzym aus der Gruppe der Heparinasen oder ein Enzym aus der Gruppe der Glucuronidasen oder eine Mischung verschiedener Enzyme aus einer der Gruppen oder eine Mischung verschiedener Enzyme aus beiden Gruppen und ein Reagenz, enthaltend Faktor Xa, enthalten sind.

12. Testkit gemäß Anspruch 11 **dadurch gekennzeichnet, dass** weiterhin ein Reagenz, enthaltend ein Faktor Xa-Substrat, enthalten ist.

13. Testkit gemäß einem der Ansprüche 11 und 12 **dadurch gekennzeichnet, dass** weiterhin ein Reagenz, enthaltend Antithrombin, enthalten ist.

14. Testkit gemäß einem der Ansprüche 11 und 12 **dadurch gekennzeichnet, dass** ein Reagenz, enthaltend ein Enzym aus der Gruppe der Heparinasen oder ein Enzym aus der Gruppe der Glucuronidasen oder eine Mischung verschiedener Enzyme aus einer der Gruppen oder eine Mischung verschiedener Enzyme aus beiden Gruppen und Antithrombin, enthalten ist.

15. Testkit gemäß den Ansprüchen 11 bis 14 **dadurch gekennzeichnet, dass** das Faktor Xa-enthaltende Reagenz zusätzlich Dextransulfat enthält.

16. Testkit gemäß den Ansprüchen 13 bis 15 **dadurch gekennzeichnet, dass** das Antithrombin-enthaltende Reagenz zusätzlich Dextransulfat enthält.

17. Testkit gemäß einem der Ansprüche 11 bis 16 **dadurch gekennzeichnet, dass** eines oder mehrere der Reagenzien lyophilisiert sind.

18. Verwendung eines Testkits gemäß einem der Ansprüche 11 bis 17 zur Durchführung eines Verfahrens gemäß Anspruch 1.

## Claims

1. Method for determining the heparin activity in a liquid sample, where the heparin-dependent inactivation of added factor Xa is quantified, **characterized in that** the sample is incubated with an enzyme from the group of heparinases or with an enzyme from the group of glucuronidases or with a mixture of different enzymes from one of the groups or with a mixture of different enzymes from both groups before addition of factor Xa.

2. Method according to Claim 1, **characterized in that** the sample is incubated with heparinase I, heparinase II, heparinase III or a mixture thereof before addition of factor Xa.

3. Method according to either of Claims 1 and 2, **characterized in that** the sample is incubated with an enzyme from the group of heparinases or with an enzyme from the group of glucuronidases or with a mixture of different enzymes from one of the groups or with a mixture of different enzymes from both groups in a concentration of from 0.05 to 5.0 U/ml, particularly preferably in a concentration of from 0.25 to 1.25 U/ml, in the mixture before addition of factor Xa.

4. Method according to any of Claims 1 to 3, **characterized in that** the sample is incubated with an enzyme from the group of heparinases or with an enzyme from the group of glucuronidases or with a mixture of different enzymes from one of the groups or with a mixture of different enzymes from both groups for 10 to 900 seconds, preferably for 15 to 90 seconds, particularly preferably for 20 to 60 seconds, before addition of factor Xa.

5. Method according to any of Claims 1 to 4, **characterized in that** the heparin-dependent inactivation of added factor Xa is quantified in the presence of added antithrombin.

6. Method according to Claim 5, **characterized in that** the antithrombin is added to the sample at the same time as an enzyme from the group of heparinases or with an enzyme from the group of glucuronidases or with a mixture of different enzymes from one of the groups or with a mixture of different enzymes from both groups.

7. Method according to any of Claims 1 to 6, **characterized in that** additionally dextran sulfate is added to the sample.

8. Method according to Claim 7, **characterized in that** the dextran sulfate is added to the sample at the same time as factor Xa.

9. Method according to any of Claims 1 to 8, **characterized in that** the heparin activity is determined in a body fluid sample, preferably in blood, blood plasma, serum or urine.

10. Method according to any of Claims 1 to 9, further **characterized in that** the heparin activity in the sample is quantified irrespective of the nature of the heparin present in the sample by means of a universal calibration curve which is suitable for all heparin variants and which has been constructed beforehand using any heparin.

11. Test kit for carrying out the method according to Claim 1, **characterized in that** a reagent comprising an enzyme from the group of heparinases or an enzyme from the group of glucuronidases or a mixture of different enzymes from one of the groups or a mixture of different enzymes from both groups and a reagent comprising factor Xa are present.

12. Test kit according to Claim 11, **characterized in that** a reagent comprising a factor Xa substrate is furthermore present.

13. Test kit according to either of Claims 11 and 12, **characterized in that** a reagent comprising antithrombin is furthermore present.

14. Test kit according to either of Claims 11 and 12, **characterized in that** a reagent comprising an enzyme from the group of heparinases or an enzyme from the group of glucuronidases or a mixture of different enzymes from one of the groups or a mixture of different enzymes from both groups and antithrombin is present.

15. Test kit according to Claims 11 to 14, **characterized in that** the factor Xa-containing reagent additionally comprises dextran sulfate.

16. Test kit according to Claims 13 to 15, **characterized in that** the antithrombin-containing reagent additionally comprises dextran sulfate.

17. Test kit according to any of Claims 11 to 16, **characterized in that** one or more of the reagents are lyophilized.

18. Use of a test kit according to any of Claims 11 to 17 for carrying out a method according to Claim 1.

## Revendications

1. Procédé de détermination de l'activité héparinique d'un échantillon liquide en quantifiant l'inactivation, qui dépend de l'héparine, de facteur Xa ajouté, **caractérisé en ce qu'**on fait incuber l'échantillon avant l'addition du facteur Xa avec un enzyme du groupe des héparinases ou avec un enzyme du groupe des glucuronidases ou avec un mélange de divers enzymes de l'un des groupes ou avec un mélange de divers enzymes des deux groupes.

2. Procédé suivant la revendication 1, **caractérisé en ce que** l'on fait incuber l'échantillon avant l'addition de facteur Xa avec de l'héparinase I, avec de l'héparinase II, avec de l'héparinase III ou avec l'un de leurs mélanges.

3. Procédé suivant l'une des revendications 1 à 2, **caractérisé en ce que** l'on fait incuber l'échantillon avant l'addition de facteur Xa avec un enzyme du groupe des héparinases ou avec un enzyme du groupe des glucuronidases ou avec un mélange de divers enzymes de l'un des groupes ou avec un mélange de divers enzymes des deux groupes, en une concentration de 0,05 à 0,5 U/ml, d'une manière particulièrement préférée en une concentration de 0,25 à 1,25 U/ml de l'addition.

4. Procédé suivant l'une des revendications 1 à 3, **caractérisé en ce qu'**on fait incuber l'échantillon avant l'addition de facteur Xa pendant 10 à 900 secondes, de préférence pendant 15 à 90 secondes, d'une manière particulièrement préférée pendant 20 à 60 secondes avec un enzyme du groupe des héparinases ou avec un enzyme du groupe des glucuronidases ou avec un mélange de divers enzymes de l'un des groupes ou avec un mélange de divers enzymes des deux groupes.

5. Procédé suivant l'une des revendications 1 à 4, **caractérisé en ce que** l'on quantifie l'inactivation, qui dépend de l'héparine, de facteur Xa ajouté en présence d'une addition d'antithrombine.

6. Procédé suivant la revendication 5, **caractérisé en ce que** l'on charge l'antithrombine en même temps qu'un enzyme du groupe des héparinases ou qu'un enzyme du groupe des glucuronidases ou qu'un mélange de divers enzymes de l'un des groupes ou qu'un mélange de divers enzymes des deux groupes.

7. Procédé suivant l'une des revendications 1 à 6, **caractérisé en ce que** l'on ajoute supplémentairement du sulfate de dextrane à l'échantillon.

8. Procédé suivant la revendication 7, **caractérisé en ce que** l'on ajoute le sulfate de dextrane à l'échantillon en même temps que le facteur Xa.

9. Procédé suivant l'une des revendications 1 à 8, **caractérisé en ce que** l'on détermine l'activité héparinique d'un échantillon de liquide corporel, de préférence du sang, du plasma sanguin, du sérum ou de l'urine.

10. Procédé suivant l'une des revendications 1 à 9, **caractérisé en outre en ce que**, indépendamment du type de l'héparine contenue dans l'échantillon, on quantifie l'activité héparinique de l'échantillon au moyen d'une courbe universelle d'étalonnage, appropriée à toutes les variantes d'héparine et établie auparavant en utilisant n'importe quelle héparine.

11. Trousse de test, pour effectuer le procédé suivant la revendication 1, **caractérisé en ce qu'**elle contient un réactif contenant un enzyme du groupe des héparinases ou un enzyme du groupe des glucuronidases ou un mélange de divers enzymes de l'un des groupes ou un mélange de divers enzymes des deux groupes et un réactif contenant du facteur Xa.

12. Trousse de test suivant la revendication 11, **caractérisé en ce qu'**elle contient en outre un réactif contenant un substrat du facteur Xa.

13. Trousse de test suivant l'une des revendications 11 et 12, **caractérisé en ce qu'**elle contient en outre un réactif contenant de l'antithrombine.

14. Trousse de test suivant l'une des revendications 11 et 12, **caractérisé en ce qu'**elle contient un réactif contenant un enzyme du groupe des héparinases ou un enzyme du groupe des glucuronidases ou un mélange de divers enzymes de l'un des groupes ou un mélange de divers enzymes des deux groupes et de l'antithrombine.

15. Trousse de test suivant l'une des revendications 11 à 14, **caractérisé en ce que** le réactif contenant du facteur Xa contient supplémentairement du sulfate de dextrane.

16. Trousse de tests suivant les revendications 13 à 15, **caractérisé en ce que** le réactif contenant de l'antithrombine contient supplémentairement du sulfate de dextrane.

17. Trousse de test suivant l'une des revendications 11 à 16, **caractérisé en ce qu'**un réactif ou plusieurs des réactifs sont lyophilisés.

18. Utilisation d'une trousse de tests suivant l'une des revendications 11 à 17, pour effectuer un procédé suivant la revendication 1.
